# EUROPÄISCHE PATENTSCHRIFT

(11) **EP 0 423 064 B1**
(45) Veröffentlichungstag und Bekanntmachung des Hinweises auf die Patenterteilung: **20.10.1993**
(21) Anmeldenummer: 90810651.1
(22) Anmeldetag: 29.08.1990
(51) Int. Cl.: A61F 2/32

(54) **Hüftgelenksprothesen mit Verankerung durch Gitterflächen**
Hip joint prosthesis with mesh-like anchoring means
Prothèse de l'articulation de la hanche pourvu de moyens d'ancrage grillagés

(30) Priorität: 12.10.1989 CH 3719/89
(43) Veröffentlichungstag der Anmeldung: 17.04.1991
(73) Patentinhaber: SULZER Medizinaltechnik AG, 8404 Winterthur (CH)
(72) Erfinder: Frey, Otto, Dr., CH-8400 Winterthur (CH); Koch, Rudolf, CH-8267 Berlingen (CH); Flückiger, Hans, CH-8128 Hinteregg (CH)
(74) Vertreter: Hammer, Bruno, Dr.

(56) Entgegenhaltungen:
- EP-A- 0 016 480
- EP-A- 0 191 182
- WO-A-89/01766
- FR-A- 2 412 304
- FR-A- 2 629 337
- GB-A- 2 024 631
- US-A- 4 888 024

## Beschreibung

Die Erfindung betrifft eine Hüftgelenkprothese mit Verankerung durch Gitterfläche, wobei die Prothese einen metallischen Trägerkörper und eine Verankerungsfläche mit einem Gitter aufweist und wobei das Gitter aus einem Geflecht besteht und vom Trägerkörper weg mindestens eine bombiert vorstehende Flache bildet. Eine solche Hüftgelenkprothese ist aus der FR-A-24 12 304 bekannt.

Bei zementfrei eingesetzten Implantaten fördern Gitterstrukturen, wie in der FR-A-24 29 589 beschrieben, an den knochenseitigen Oberflächen das Einwachsen von Knochengewebe. Eine zementfrei durch Verformung von unter Innendruck stehenden Blechhohlkörpern erzeugte Verankerung ist in der EP-A-0 191 182 aufgeführt und die EP-A-0 016 480 zeigt einen losen, aus mehreren Schichten bestehenden Strumpf, der in die vorbereitete Kavität eines Femurknochens eingelegt und innen mit Knochenzement gefüllt wird, um ein Zementbett für eine Femurkopfprothese zu bilden. Der Strumpf ist auf der Innenseite flüssigkeitsdicht ausgeführt, damit er unter Innendruck auch an entfernteren Hohlräumen anliegt. Eine Armierung vom Zement findet nicht statt.

Für unter Knochenkontakt einzementierte Prothesen zeigt die FR-A-24 12 304 Drahtgitter, die auf dem Schaft der Prothese aufgezogen sind und mit der Prothese in ein Zementbett gesetzt werden. Die Armierung ist hier nur in ihrer vorher bestimmten Dicke und bezüglich der Trennschicht zwischen Knochen und Zement nur an wenigen Auflagepunkten wirksam.

Hier schafft die Erfindung Abhilfe. Sie hat die Aufgabe, eine innige Verzahnung von einwachsendem Knochengewebe mit dem prothesenseitigen Zement über grosse Flächen herzustellen und auch hinterschnittene Knochenaushöhlungen mit Zement und einer Armierung auszufüllen.

Diese Aufgabe wird mit den kennzeichnenden Merkmalen des unabhängigen Anspruchs 1 gelöst.

Die Vorteile der Erfindung sind darin zu sehen, dass sich die Prothese beim Einbringen den spezifischen Knochenaushöhlungen anpasst, ohne bestimmte Referenz-Auflagepunkte zu verändern, um dann in dieser Lage fixiert zu werden.

Im folgenden wird die Erfindung anhand von Ausführungsbeispielen beschrieben. Es zeigen:
- Fig. 1: die Seitenansicht einer eingesetzten Femurkopfprothese beim Füllen der Hohlräume unter den Gitterflächen mit Knochenzement;
- Fig. 2: einen Querschnitt durch den eingesetzten Prothesenschaft in Fig. 1;
- Fig. 3: die Seitenansicht einer Femurkopfprothese vor dem Implantieren;
- Fig. 4: einen Querschnitt durch den Prothesenschaft in Fig. 3;
- Fig. 5: den Längsschnitt durch eine eingesetzten Hüftgelenksschale beim Füllen der Hohlräume unter den Gitterflächen mit Knochenzement;
- Fig. 6: einen Längsschnitt durch das Gitter der Hüftgelenksschale von Fig. 5 vor dem Implantieren.

In den Figuren sind Hüftgelenksprothesen mit Verankerung durch Gitterflächen gezeigt, wobei die Prothesen einen metallischen Trägerkörper 1, 11 mit Verankerungsflächen 3 mit einem Gitter 4 aufweisen. Erfindungsgemäss besteht das Gitter 4 aus einem Geflecht und bildet vom Trägerkörper 1, 11 weg nach aussen eine oder mehrere bombiert vorstehende Flächen 10, die an ihren Rändern mit dem Trägerkörper 1, 11 verbunden sind und die unter nach innen angreifenden Einzelkräften nach innen elastisch deformierbar sind. Unter dem Gitter 4 sind Hohlräume 5 ausgenommen, welche die Deformation des Gitters nach innen zum Trägerkörper hin zulassen und welche im eingesetzten Zustand des Implantats über Einfüllöffnungen 6, 13, 14 mit Knochenzement stopfbar sind.

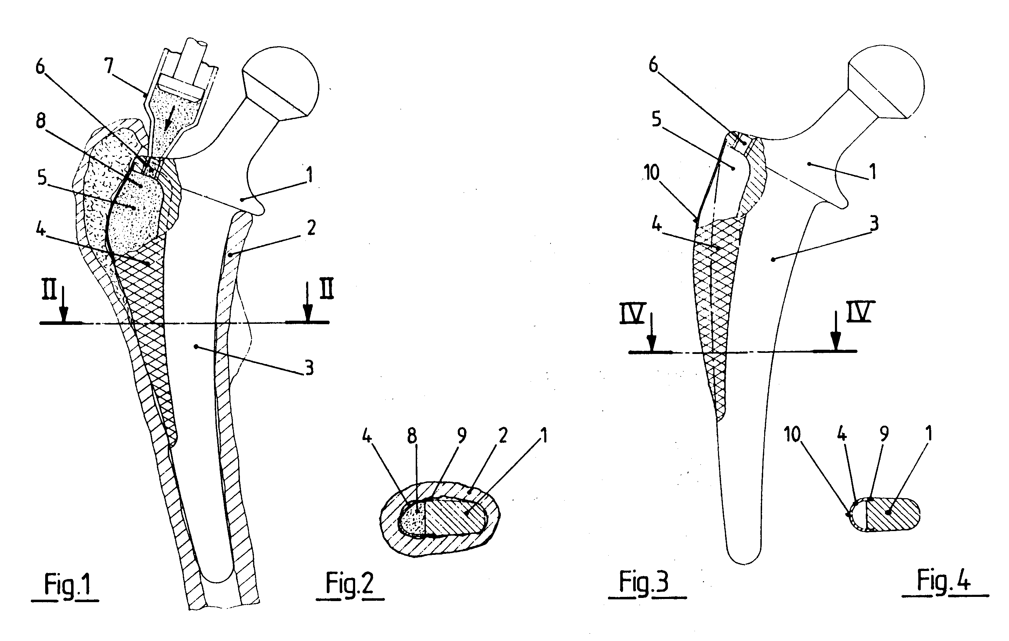

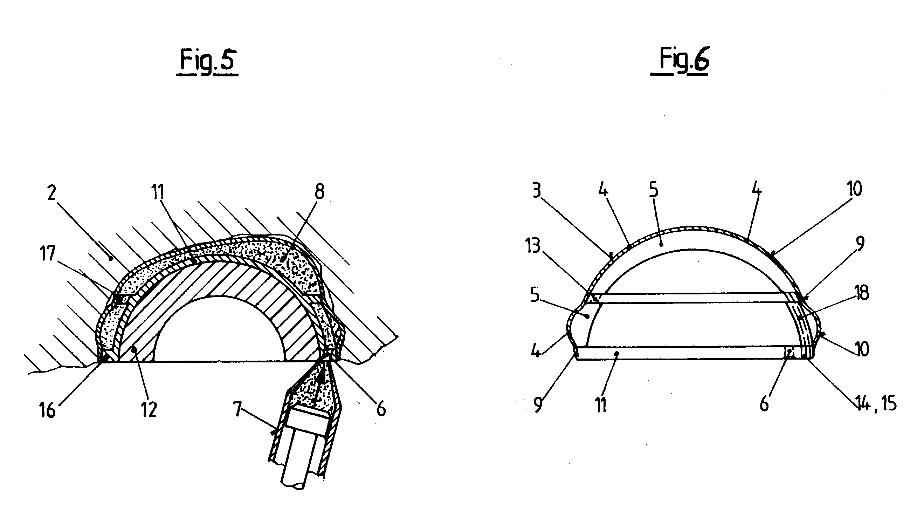

Die Fixierung der Gitter 4 an den Trägerkörpern 1, 11 kann durch eine Gefügebindung, z.B. durch Schweissen, erfolgen. Beim Einbringen des Implantats in eine vorbereitete Kavität im Knochen 2 federn die bombiert vorstehenden Flächen 10 des Gitters 4 beim Auftreffen auf Hindernisse zurück und folgen der Wand der Knochenaushöhlung. Dabei legt sich das Gitter 4 in grossen Bereichen an den Knochen 2 an. Dieses Anliegen wird verstärkt, wenn das Gitter 4 für pasteuse Massen weitgehend undurchlässig ist und Knochenzement durch vorgesehene Einfüllöffnungen 6, 14 in die Hohlräume 5 unter den Gittern 4 eingespritzt wird. Das Gitter 4 folgt dann der individuellen Form der Kavität im Knochen und ist auf der Innenseite bis in seine innere Struktur mit dem ausgehärteten Knochenzement verzahnt, während von der Knochenseite frisches Knochengewebe über die engen Restspalten in die verbleibende Gitterstruktur einwachsen kann. Das Gitter 4 übernimmt damit die Funktion einer Kupplung zwischen Knochengewebe 2 und Zement 8, die Druckkräfte und Kräfte tangential zum Gitter 4 überträgt. Die deformierbaren Gitterflächen 10 sind so angeordnet, dass sie Hinterschneidungen in den Knochenkavitäten ausfüllen und die Prothese quer zur Ausziehrichtung verkeilen. Bei dem Prothesenschaft in Fig. 1 und 3 liegt eine sich anpassende Gitterfläche 10 in Richtung vom grossen Trochanter, während die Verankerungsfläche 3 auf der Gegenseite des Schaftes 1 eine unveränderte Referenzfläche für die Ausrichtung bildet. Bei der Hüftgelenksschale in Fig. 5 und 6 bildet eine Ringschulter 17 die Referenzauflage für die Ausrichtung der Trägerschale 11 während des Füllens des Ringraumes zwischen Ringschultern 16 und 17 mit Knochenzement. Der Scheitelraum zwischen Gitter 4 und Trägerschale 11 wird über Verbindungsöffnungen 13, 14, 15 gefüllt respektive entlüftet. Um diesen Raum vom Rand der Trägerschale 11 her zu erreichen, sind die Verbindungsöffnungen 14, 15 in Verbindungsstegen 18 untergebracht. Um die Füllung der Hohlräume 5 sicherzustellen, wird der Knochenzement mit einem bestimmten Druck eingespritzt, wobei eine Einspritzvorrichtung 7 mit den Einfüllöffnungen 6, 14 koppelbar ist. Statt des heute üblichen Knochenzements können auch elastischere Stoffe zum Ausfüllen der Hohlräume 5 eingesetzt werden, solange sie körperverträglich sind und als pasteuse Masse eingefüllt werden können.

## Patentansprüche

1. Hüftgelenkprothese mit Verankerung durch Gitterfläche, wobei die Prothese einen metallischen Trägerkörper (1, 11) und eine Verankerungsfläche mit einem Gitter (4) aufweist und wobei das Gitter (4) aus einem Geflecht besteht und vom Trägerkörper (1, 11) weg nach aussen mindestens eine bombiert vorstehende Fläche (10) bildet, dadurch gekennzeichnet, dass das Gitter (4) an seinen Rändern mit dem Trägerkörper (1, 11) verbunden ist und unter nach innen angreifenden Einzelkräften nach innen elastisch deformierbar ist, dass das Gitter (4) für eine pasteuse Masse weitgehend undurchlässig ist und dass unter dem Gitter (4) wenigstens ein Hohlraum (5) ausgenommen ist, welcher die Deformation des Gitters (4) nach Innen zum Trägerkörper hin zulässt und welcher bei im Körper positionierter Prothese über wenigstens eine Einfüllöffnung (6, 13, 14) an der Prothese mit Knochensement (8) stopfbar ist.

2. Hüftgelenkprothese nach Anpruch 1, dadurch gekennzeichnet, dass das Gitter (4) über Gefügebindungen mit dem Trägerkörper (1, 11) verbunden ist.

3. Hüftgelenkprothese nach Anspruch 1 oder 2, dadurch gekennzeichnet, dass beim Einbringen des Knochenzements (8) die Verdrängung der Luft aus den Hohlräumen (5) nach aussen durch das Gitter (4) hindurch oder durch verschliessbare Entlüftungsöffnungen (15) erfolgt.

4. Hüftgelenkprothese nach einem der Ansprüche 1 bis 3, dadurch gekennzeichnet, dass die bombierte Gitterfläche (10) geometrisch so angeordnet ist, dass mit wachsendem Einspritzdruck vom Knochenzement (8) in die Hohlräume (5) die Haftung vom Gitter (4) im Knochengewebe vergrössert wird, indem die Deformationsmöglichkeiten quer zur Einbringrichtung der Prothese vorgesehen sind.

5. Hüftgelenkprothese nach einem der Ansprüche 1 bis 4, dadurch gekennzeichnet, dass die Einfüllöffnung (6, 14) eine Kupplungsmöglichkeit zu einer Einspritzvorrichtung (7) aufweist.

6. Hüftgelenkprothese nach einem der Ansprüche 1 bis 5, dadurch gekennzeichnet,dass sie eine Einspritzvorrichtung aufweist, die einen einstellbaren Druck bis zum Aushärten des Knochenzements erzeugt.

7. Hüftgelenkprothese nach einem der Ansprüche 1 bis 5, dadurch gekennzeichnet, dass sie eine Femurkopfprothese ist und dass das Gitter (4) und der zugehörige Hohlraum (5) auf der Seite zum grossen Trochanter hin angeordnet sind.

8. Hüftgelenkprothese nach einem der Ansprüche 1 bis 5, dadurch gekennzeichnet, dass sie eine künstliche Hüftpfanne ist und dass die bombierte Fläche (10) mindestens Segmente eines äusseren Ringes am Rand einer Trägerschale (11) abdeckt.

9. Hüftgelenkprothese nach Anspruch 8, dadurch gekennzeichnet, dass Verbindungsstege (18) mit Einfüllöffnungen (14) und/oder Entlüftungsöffnungen (15) vom stirnseitigen Rand zum Raum über dem Scheitel der Trägerschale (11) angebracht sind.

## Claims

1. A hip joint prosthesis secured by a mesh surface, whereby the prosthesis comprises a metal supporting member (1, 11) and an attachment surface having a mesh (4) and whereby the mesh (4) consists of a network and in the outward direction away from the supporting member (1, 11) forms at least one dished projecting surface (10),
**characterised in that** at its edges the mesh (4) is connected to the supporting member (1, 11) and is elastically deformable in the inward direction under inwardly acting concentrated forces,
**in that** the mesh (4) is largely impermeable to a pasty substance,
**in that** under the mesh (4) is provided at least one cavity (5), which permits the inward deformation of the mesh (4) towards the supporting member and which, when the prosthesis is positioned in the body, can be filled with bone cement (8) via at least one feed aperture (6, 13, 14) on the prosthesis.

2. A hip joint prosthesis according to Claim 1,
**characterised in that** the mesh (4) is connected to the supporting member (1, 11) via structural bonds.

3. A hip joint prosthesis according to Claim 1 or 2,
**characterised in that** when inserting the bone cement (8), air is expelled from the cavities (5) towards the outside through the mesh (4) or through sealable vents (15).

4. A hip joint prosthesis according to one of Claims 1 to 3,
**characterised in that** the dished mesh surface (10) is geometrically arranged so that as the pressure at which bone cement (8) is injected into the cavities (5) increases, the adhesion of the mesh (4) in the osseous tissue is increased by the deformation facilities being provided at right angles to the direction of insertion of the implant.

5. A hip joint prosthesis according to one of Claims 1 to 4,
**characterised in that** the feed aperture (6, 14) comprises a coupling facility to an injection device (7).

6. A hip joint prosthesis according to one of Claims 1 to 5,
**characterised in that** it comprises an injection device which produces an adjustable pressure until the bone cement has hardened.

7. A hip joint prosthesis according to one of Claims 1 to 5,
**characterised in that** it is a femur head prosthesis, **and in that** the mesh (4) and the associated cavity (5) are disposed on the side facing the greater trochanter.

8. A hip joint prosthesis according to one of Claims 1 to 5,
**characterised in that** it is an artificial acetabulum, **and in that** the dished surface (10) covers at least segments of an outer ring on the edge of a supporting shell (1).

9. A hip joint prosthesis according to Claim 8,
**characterised in that** connecting flanges (18) with feed apertures (14) and/or vents (15) are mounted from the front edge to the space above the apex of the supporting shell (11).

## Revendications

1. Prothèse de l'articulation coxo-fémorale pourvue d'un treillis d'ancrage, la prothèse comportant un corps porteur métallique (1, 11) et une surface d'ancrage avec un treillis (4) et le treillis (4) étant constitué d'une maille et formant en partant du corps porteur (1, 11) vers l'extérieur au moins une surface saillante bombée (10), caractérisée en ce que le treillis (4) est lié à ses bords au corps porteur (1, 11) et est élastiquement déformable vers l'intérieur sous l'influence de forces isolées agissant vers l'intérieur, en ce que le treillis (4) est largement imperméable à une substance pâteuse et en ce que sous le treillis (4) est réalisée au moins une cavité (5), qui permet la déformation du treillis (4) vers l'intérieur vers le corps porteur et qui, dans le cas d'une prothèse placée dans le corps, peut être obturée, par au moins un orifice de remplissage (6, 13, 14) situé sur la prothèse, avec du ciment pour os (8).

2. Prothèse de l'articulation coxo-fémorale suivant la revendication 1, caractérisée en ce que le treillis (4) est lié par des liaisons d'assemblage au corps porteur (1, 11).

3. Prothèse de l'articulation coxo-fémorale suivant la revendication 1 ou 2, caractérisée en ce que , lors de la mise en place du ciment pour os (8), le déplacement de l'air des cavités (5) vers l'extérieur est réalisé à travers le treillis (4) ou à travers des orifices d'aération (15) pouvant être obturés.

4. Prothèse de l'articulation coxo-fémorale suivant l'une des revendications 1 à 3, caractérisée en ce que la surface en treillis bombée (10) est géométriquement conçue de telle façon qu'avec une pression d'injection croissante du ciment pour os (8) dans les cavités (5), l'adhérence du treillis (4) dans le tissu osseux est augmentée, en prévoyant les possibilités de déformation transversalement par rapport au sens de mise en place de la prothèse.

5. Prothèse de l'articulation coxo-fémorale suivant l'une des revendications 1 à 4, caractérisée en ce que l'orifice de remplissage (6, 14) dispose d'une possibilité de couplage pour un dispositif d'injection (7).

6. Prothèse de l'articulation coxo-fémorale suivant l'une des revendications 1 à 5, caractérisée en ce qu'elle dispose d'un dispositif d'injection qui produit une pression réglable jusqu'à la prise du ciment pour os.

7. Prothèse de l'articulation coxo-fémorale suivant l'une des revendications 1 à 5, caractérisée en ce qu'elle est une prothèse de tête fémorale et en ce que le treillis (4) et la cavité afférente (5) sont disposés sur la face côté grand trochanter.

8. Prothèse de l'articulation coxo-fémorale suivant l'une des revendications 1 à 5, caractérisée en ce qu'elle est un cotyle artificiel et en ce que la surface bombée (10) couvre au moins des segments d'un anneau extérieur sur le bord d'une coque de support (11).

9. Prothèse de l'articulation coxo-fémorale suivant la revendication 8, caractérisée en ce que des passerelles de liaison (18), comportant des orifices de remplissage (14) et/ou des orifices d'aération (15), sont disposées du bord côté frontal jusqu'à l'espace situé au-dessus du point culminant de la coque de support (11).
